(19) Europäisches Patentamt

European Patent Office

Office européen des brevets

(11) **EP 1 136 089 B1**

(12) **FASCICULE DE BREVET EUROPEEN**

(45) Date de publication et mention
de la délivrance du brevet:
**22.02.2006 Bulletin 2006/08**

(51) Int Cl.:
*A61M 5/168* *(2006.01)*

(21) Numéro de dépôt: **01106188.4**

(22) Date de dépôt: **14.03.2001**

(54) **Procédé d'analyse de la variation de pression dans un dispositif de perfusion comprenant plusieurs modules de perfusion**

Verfahren zur Analyse von Druckänderungen in einem Infusionsvorrichtung mit mehrerer Infusionsmodule

Method for analysing varying pressure in an infusion device having multiple infusion modules

(84) Etats contractants désignés:
**AT BE CH CY DE DK ES FI FR GB GR IE IT LI LU MC NL PT SE TR**

(30) Priorité: **16.03.2000 FR 0003509**

(43) Date de publication de la demande:
**26.09.2001 Bulletin 2001/39**

(73) Titulaire: **Fresenius Vial SAS**
**38590 Brézins (FR)**

(72) Inventeurs:
• **Wolff, Rémi**
**38210 Morette (FR)**

• **Rondelet, Jean-Claude**
**38960 Saint Etienne de Crossey (FR)**

(74) Mandataire: **Vièl, Christof**
**Cabinet Vièl**
**1, rue des Bleuets,**
**BP 18**
**57520 Grosbliederstroff (FR)**

(56) Documents cités:
**EP-A- 0 960 627        DE-C- 19 642 234**
**FR-A- 2 710 537        US-A- 5 464 392**
**US-A- 5 647 853**

EP 1 136 089 B1

**Description**

L'invention concerne un procédé, d'analyse de la variation de la pression dans un dispositif de perfusion comprenant plusieurs modules de perfusion munis chacun d'une pompe pour entraîner un liquide à perfuser dans une ligne placée en aval de la pompe ainsi que d'un moyen de mesure de la pression dans la ligne, des points de jonction permettant de raccorder certaines lignes entre elles ou certaines lignes avec des lignes provenant d'unités externes au dispositif de perfusion ainsi qu'un dispositif de perfusion pour la mise en oeuvre du procédé.

Les dispositifs de perfusion comportent habituellement une source de liquide reliée à un tube souple qui se prolonge par une canule ou un cathéter destiné à être inséré dans le corps du patient. Afin d'assurer un débit contrôlé du liquide, il est courant de placer une pompe le long du tube. Cette pompe, lorsqu'elle est du type pousse seringue, contient également la source de liquide.

Lorsque plusieurs produits sont à injecter, il est parfois nécessaire de faire appel à plusieurs de ces unités de perfusion. Les lignes sortant de certaines pompes peuvent être raccordées entre elles pour permettre un mélange de différents produits. Les différentes unités sont munies de moyens de contrôle qui peuvent déclencher des alarmes, voire même interrompre la perfusion lorsque certains critères de contrôle sont vérifiés.

Le bon déroulement de la perfusion peut, dans certains cas, être vital. Il est donc impératif que le produit soit administré conformément au plan d'administration prévu.

Or, il arrive que des événements se produisent qui perturbent le déroulement de la perfusion d'un des produits. Ces évènements sont de trois sortes :

- une occlusion dans la ligne en aval de la pompe ;
- une rupture de la ligne en aval ou en amont de la pompe, ou une occlusion en amont de la pompe ;
- une variation du débit dans une pompe dont la ligne est raccordée par un point de jonction avec d'autres lignes (par exemple en cas de bolus).

Ces événements se traduisent tous par une variation de la pression dans la ligne considérée. C'est ainsi que l'on constatera une augmentation de la pression dans la ligne k par exemple dans les cas suivants :

- occlusion dans la ligne k ;
- augmentation du débit, par exemple dans le cadre d'un bolus, dans une ligne j raccordée à la ligne k par un point de jonction.

On constatera au contraire une diminution de la pression par exemple dans les cas suivants :

- rupture de la ligne;
- diminution du débit dans une ligne j raccordée à la ligne k par un point de jonction ;

Dans les dispositifs connus, chaque unité de perfusion, ou module, est en général munie de moyens pour contrôler et analyser les variations de pression afin de déclencher des alarmes et le cas échéant interrompre la perfusion. C'est ainsi que si la pression mesurée dans la ligne dépasse une certaine valeur, une alarme est déclenchée et la pompe du module concerné est arrêtée. L'utilisateur, en général un membre du personnel médical, doit ensuite déterminer la cause de l'augmentation anormale de la pression. Si l'augmentation n'a pas d'explication, par exemple cette augmentation de la pression n'est pas due à un bolus manuel, il faut en conclure qu'une occlusion est apparue en aval de la pompe. Cependant, depuis l'apparition de l'occlusion, la pompe a continué de pomper jusqu'à ce que la pression dans la ligne atteigne une valeur d'alarme. Si l'occlusion est supprimée subitement, tout le liquide placé en surpression en aval de la pompe et qui aurait dû être administré en un certain laps de temps est délivré brusquement. Afin d'empêcher un tel phénomène, la pompe concernée par l'occlusion est arrêtée puis mise en marche arrière pour aspirer la quantité de liquide libérée depuis la formation de l'occlusion, de façon à éliminer complètement le bolus. C'est seulement à ce moment là que l'occlusion peut être éliminée sans danger.

Ce système de détection individuelle de la pression pour chaque ligne issue d'une pompe est très efficace tant que la ligne considérée n'est pas raccordée à une autre ligne. Cependant, dans les dispositifs de perfusion à plusieurs unités, il n'est pas rare que certaines lignes se rejoignent en un point de jonction. Si une occlusion se produit en aval d'un tel point de jonction, la pompe dont le seuil d'alarme est le plus bas émettra en premier un signal d'alarme et commencera à pomper en marche arrière jusqu'à la disparition de la surpression dans sa ligne. Cependant, les autres pompes dont les lignes sont raccordées au point de jonction n'ont pas encore détecté d'occlusion puisque leurs seuils d'alarme ne sont pas encore atteints. Elles continuent donc de pomper normalement, alimentant la surpression. La pompe qui se trouve en position de marche arrière va donc non seulement aspirer ce qu'elle a délivré depuis l'apparition de l'occlusion, mais elle va en plus aspirer le liquide délivré par les autres pompes auxquelles elle est raccordée par le point de jonction. Le liquide contenu ainsi dans la pompe mise en marche arrière devient incertain et ne peut plus être utilisé sans risque pour le patient.

La même augmentation de pression, qui a provoqué l'arrêt de la pompe k, peut être due non pas à une occlusion mais à une augmentation passagère (cas du bolus) ou durable du débit dans une ligne j raccordée à la ligne ayant détecté l'augmentation anormale de la pression. Le module k et la perfusion auront donc été interrompus inutilement.

L'objectif de l'invention est donc de mettre au point un procédé qui permette d'éviter les dysfonctionnements dus au fait que certaines lignes peuvent être raccordées les unes aux autres.

Cet objectif est atteint par le procédé conforme à l'invention dans lequel, lorsqu'une variation de pression Pk dans une ligne k est détectée, une procédure d'analyse est mise en oeuvre pour déterminer l'implication d'autres modules j dans cette variation de pression. Cette procédure permet de tenir compte de l'environnement du module concerné avant d'agir, en tenant compte d'informations provenant soit d'un autre module soit d'une information externe. Pour cela, les modules doivent pouvoir communiquer soit entre eux soit avec une base regroupant l'ensemble des données et qui les retransmet aux modules pouvant être concernés par l'information. La procédure d'analyse est lancée aussi bien en cas d'augmentation qu'en cas de diminution de la pression. Elle a pour objectif d'analyser la situation environnementale du module concerné afin, selon les résultats fournis par cette analyse doit d'éviter une interruption inutile de la perfusion si la cause de cette variation est expliquée (variation de débit dans un module raccordé par un point de jonction, bolus manuel à un point de jonction avec la ligne concernée) soit d'arrêter simultanément toutes les pompes concernées par une occlusion ou une rupture.

Dans une première variante, la procédure inclut une recherche d'informations indiquant une modification du débit dans un autre module j. Lorsque le débit dans une ligne est modifié, le module concerné émet, automatiquement ou sur demande, une information concernant les modifications effectuées. Si un module k détecte une variation de pression dans sa ligne, il recherchera une telle information. S'il en trouve une, il en conclura que la variation de pression a une explication connue.

Dans un développement supplémentaire de cette première variante, la procédure d'analyse prévoit, lorsqu'une information indiquant une modification de débit dans un autre module j a été trouvée, de modifier les paramètres d'analyse du module k au moins le temps que dure la modification du débit dans le module j. C'est ainsi que si le module k constate que le débit a été augmenté dans un second module, il modifie ses paramètres d'analyse pour ne plus être en situation d'alarme. Cette modification peut être de courte durée, par exemple le temps d'un bolus, ou durable, si l'augmentation du débit est prolongée. L'intérêt de cette procédure est d'éviter d'arrêter la perfusion inutilement.

Selon une seconde variante, la procédure d'analyse inclut la comparaison de la pente de la courbe de pression de chaque ligne i du système avec la pente de la courbe de pression de la ligne k pour déterminer les lignes j qui sont potentiellement raccordées à la ligne k par un point de jonction et qui peuvent être également concernées par la variation de pression. Si dans une ligne j, on constate une augmentation de la pression semblable à l'augmentation de la pression dans la ligne k, il est probable que la ligne j est raccordée à la ligne k par un point de jonction. En raison des pertes de charge et des différents moyens de mesure de la pression pouvant être utilisés, il est possible que les variations de pression dans les lignes j et la ligne k ne soient pas tout à fait identiques. On pourra par exemple fixer deux seuils de tolérance qui indiqueront lorsqu'ils sont atteints que les lignes sont sûrement raccordées ou qu'elles ne sont que probablement raccordées entre elles. Cette information permettra de mieux estimer les interactions de ces modules entre eux pour analyser plus précisément les variations de pression dans une ligne k.

Une troisième variante prévoit d'inclure dans la procédure d'analyse la comparaison de la vitesse d'augmentation de la pression dans la ligne k avec la vitesse théorique qu'elle devrait avoir si une occlusion se produisait dans la ligne en amont de tout point de jonction avec une autre ligne. Si le module est utilisé seul, c'est-à-dire sans raccordement avec d'autres lignes, il est possible, en fonction du débit et de la compliance des éléments le constituant, de déterminer la vitesse théorique d'augmentation de la pression en cas d'occlusion dans la ligne. Si la ligne k dans laquelle une augmentation de pression a été détectée est raccordée à d'autres lignes, la vitesse d'augmentation de la pression dans le module k ne correspondra pas à la vitesse théorique calculée pour le même module considéré seul. Par contre, si cette vitesse est semblable à la vitesse théorique, il est très probable que l'occlusion soit située en amont de tout point de jonction.

Ces trois variantes de réalisation de la procédure d'analyse permettent d'une part de rechercher une explication admissible à une variation de pression et d'autre part de déterminer si le module dans lequel la variation de pression a été détectée est raccordé à d'autres modules, et dans l'affirmative quels sont ces modules. Cependant, ces variantes de réalisation ne donnent qu'un début d'information qui parfois est insuffisant. Il est donc préférable de combiner ces variantes afin d'affiner l'analyse.

C'est ainsi que dans une sous-variante combinant la seconde et la troisième variante, la procédure d'analyse inclut le calcul de la vitesse théorique d'augmentation de la pression qui devrait être observée dans la ligne k en cas d'occlusion en aval des points de jonction avec les lignes j et la comparaison de la vitesse d'augmentation réelle dans la ligne k avec cette vitesse théorique. Si la procédure selon la troisième variante montre que le module ne peut pas être considéré comme isolé (par conséquent qu'il n'y a pas d'occlusion en amont de tout point de jonction avec d'autres modules), la procédure selon la seconde variante est mise en oeuvre pour déterminer quels modules sont concernés par cette variation de pression, c'est-à-dire ceux qui sont raccordés à la ligne k par un point de jonction. Quand on sait quels sont les autres modules concernés, on calcule à l'aide de leurs débits et de leurs paramètres de compliance respectifs la vitesse théorique d'augmentation de la pression dans la ligne k si une occlusion était située en aval du point de jonction de ces modules. Si la vitesse réelle correspond à cette nouvelle vitesse théorique, on peut en conclure qu'il s'est produit une occlusion en aval du point de jonction. Dans le cas contraire, il faut en conclure que la variation de pression est vraisemblablement due à une variation de débit dans un module raccordé au module k ou à un bolus manuel à un point de jonction situé sur la ligne k. Cette hypothèse peut être vérifiée par exemple en effectuant la procédure selon la première variante.

Du point de vue pratique, il est préférable de mesurer à intervalles réguliers la pression Pi dans chaque ligne i, et de mémoriser ces mesures dans un fichier historique au plus tard à partir du moment où une variation de pression est détectée dans une ligne k. Pour certaines applications, il est préférable de commencer la mémorisation des valeurs dès le début de la perfusion afin de pouvoir déterminer, si une occlusion s'est produite, le moment où celle-ci est apparue. Il n'est pas forcément nécessaire de conserver les enregistrements pendant toute la durée de la perfusion. Pour des raisons d'économie de mémoire, il peut être préférable de ne garder les informations que pendant un certain laps de temps. Ce laps de temps sera déterminé en fonction du débit des différentes pompes et donc du temps nécessaire pour détecter une occlusion.

Il est conforme à l'invention que la procédure d'analyse soit lancée lorsque la pression Pk dans une ligne k atteint une valeur seuil fixée pour chaque pompe. Dans la pratique on fixera pour chaque module un seuil inférieur et un seuil supérieur, la pression normale devant être située entre ces deux valeurs. Dès que la pression sort de ce domaine de tolérance, la procédure d'analyse est lancée. Il est possible de fixer une seconde paire de valeurs seuils comprise dans le domaine précédent, qui lance une procédure d'observation, par exemple en enregistrant les valeurs mesurées.

Lorsque les résultats de la procédure d'analyse montrent que la variation de pression est due soit à une rupture dans une ligne (diminution de la pression dans une ou plusieurs lignes) soit à une occlusion dans une ligne (augmentation de la pression non expliquée dans une ou plusieurs lignes), il est nécessaire d'agir au plus vite. C'est pourquoi le procédé prévoit, lorsque les résultats de la procédure d'analyse amènent à conclure qu'une rupture ou une occlusion s'est produite en aval de la pompe k, d'arrêter la pompe k ainsi que, le cas échéant, les pompes j raccordées à la pompe k par leurs lignes respectives en des points de jonction situés en amont de la rupture ou de l'occlusion. On n'arrête donc pas uniquement la pompe k qui a déclenché l'alarme, mais également les pompes j reliées à la pompe k par un point de jonction situé en amont de l'occlusion ou de la rupture. En cas d'occlusion, on évite ainsi d'augmenter la surpression en amont de l'occlusion en laissant pomper chaque pompe j jusqu'à ce que sa valeur seuil d'alarme soit à son tour atteinte. Par ailleurs, si on n'arrête pas les autres modules k, les liquides fournis par ces modules auront tendance sous l'effet de la pression à refluer dans la ligne k, provoquant un mélange indéterminé. Cet inconvénient est évité en arrêtant simultanément tous les modules situés en amont de l'occlusion.

Afin d'éviter le phénomène de bolus lorsque l'occlusion est supprimée, il est conforme à l'invention de mettre en marche arrière chaque pompe j qui a été arrêtée pendant un laps de temps $\Delta tj$, à un débit inverse RQj proportionnel au débit initial Qj lors du fonctionnement normal. Pour cela on détermine par exemple parmi les pompes j concernées par l'occlusion la pompe s qui avait le débit de pompage Qs le plus élevé, on fait pomper cette pompe s à l'envers à un débit inverse RQs et les autres pompes j au débit inverse respectif

$$RQj = (Qj/Qs) \times RQs.$$

Le laps de temps $\Delta tj$ sera choisi de sorte que d'une part on évite un bolus et d'autre part il n'y ait pas d'aspiration de sang.

Dans une première variante de réalisation de ce procédé, les laps de temps $\Delta tj$, pendant lesquels les pompes concernées par l'occlusion pompent en marche arrière au débit inverse RQj, sont choisis identiques pour toutes lesdites pompes et valent

$$\Delta t = (T2 - T0) \times \sum(Qj) / \sum (RQj),$$

où T0 est l'instant auquel l'occlusion est apparue, cet instant T0 étant déterminé au moyen de l'historique des mesures enregistrées dès le début de la perfusion, et T2 est l'instant où l'occlusion a été détectée. Pour appliquer cette variante, il est donc nécessaire de commencer l'enregistrement des données concernant les mesures de pression dès le début de la perfusion et il faut les conserver assez longtemps afin de pouvoir déterminer l'instant T0 auquel l'occlusion s'est formée.

Dans une seconde variante de réalisation de ce procédé, chaque pompe j concernée par l'occlusion pompe à l'envers jusqu'à ce que la pression déterminée dans sa ligne soit passée en dessous d'un seuil Plj fixé. Pour cette variante de réalisation, il n'est donc pas nécessaire de déterminer préalablement un laps de temps particulier $\Delta$tj, le contrôle de la pression dans chaque tube j étant suffisant pour arrêter les pompes concernées.

Afin de faciliter le travail du personnel médical, le résultat de la procédure d'analyse est représenté sous la forme d'un schéma de raccordement des différentes lignes, par exemple sur un écran intégré à la base.

L'objectif de l'invention est atteint également par le fait que le dispositif de perfusion à plusieurs pompes est muni d'un dispositif de mise en oeuvre du procédé selon l'invention. Ce dispositif de mise en oeuvre du procédé peut être placé soit directement sur chaque pompe si les pompes sont capables de communiquer entre elles, soit dans un dispositif de contrôle externe relié à chaque pompe, tel qu'un ordinateur ou une base.

Des exemples de réalisation de l'invention sont décrits ci-dessous à l'aide de figures et de schémas de principe joints.

La figure 1     représente un dispositif de perfusion à sept modules dont certains sont reliés par des points de jonction ;

La figure 2     une courbe de pression dans le module k au cours d'une augmentation de débit dans un module j raccordé ;

La figure 3     est un schéma de principe d'une seconde variante de réalisation de la procédure d'analyse ;

La figure 4     est un schéma de principe d'une troisième variante de réalisation de la procédure d'analyse ;

La figure 5     est un schéma de principe d'une première procédure de pompage en marche arrière des pompes concernées par l'occlusion ;

La figure 6     est un schéma de principe d'une seconde variante de procédure de pompage en marche arrière des pompes concernées par l'occlusion.

Le dispositif de perfusion auquel doit être appliqué le procédé objet de l'invention comporte plusieurs modules (1 à 7). Ces modules (1 à 7) comportent chacun une pompe entraînant un liquide à perfuser dans une ligne (11 à 17) et un capteur de pression indiquant la pression dans la ligne correspondante. Les pompes peuvent être soit des pompes volumétriques, auquel cas elles sont alimentées par une source de liquide extérieure, soit des pompes de type pousse seringue, auquel cas, la source de liquide est la seringue elle-même. Les détecteurs de pression peuvent être placés en aval de la pompe, ce sera le cas en général pour les pompes volumétriques. Pour les pousses seringue, les détecteurs peuvent être placés sur l'organe actionnant la seringue. Des points de jonction (31, 32, 33, 34) permettent de raccorder certaines lignes entre elles. Les modules sont reliés à une base (25) qui centralise les informations en provenance et à destination des différents modules. Elle est capable également de gérer des informations provenant de l'extérieur. Au lieu de faire appel à une base, il est également possible que chaque module puisse communiquer directement avec les autres modules.

Avant de commencer la perfusion, certains paramètres sont fixés pour chaque module, en fonction du produit qu'il a à délivrer. C'est ainsi que sont fixés pour chaque module i :

•   le débit Qi ;
•   les paramètres d'analyse de chaque module i, à savoir les pressions limite supérieures P1i et P2i et les pressions limite inférieures P3i et P4i ; les paramètres P1i et P3i définissent le domaine normale de variation de pression et les paramètres P2i et P4i les valeurs qui lorsqu'elles sont atteintes déclenche une action ;
•   les pressions limite PLi qui ne doivent en aucun cas être dépassées (on pourra par exemple fixer PLi = P2i).

Pour simplifier les choses, on affectera par la suite un indice

•   i aux paramètres qui doivent s'appliquer à toutes les pompes :

•   j aux paramètres qui n'intéressent que les pompes concernées par une occlusion ;
•   k aux paramètres concernant la pompe ayant détecté une variation de pression ;
•   s aux paramètres concernant la pompe ayant le débit Qj le plus élevé parmi les pompes j concernées par une occlusion.

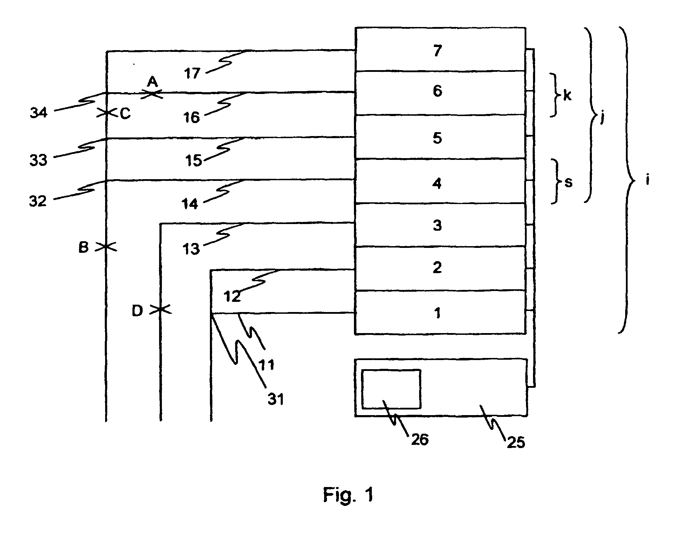

Ces indices sont illustrés à la figure 1. L'occlusion s'est produite en B, le module (6) a détecté une variation de pression, le module (4) est, parmi les modules j (4 à 7) concernés par l'occlusion, celui dont le débit Qs est le plus élevé.

La perfusion est commencée et la pression dans les lignes i (11 à 17) est mesurée, par exemple toutes les 10 secondes. Ces valeurs sont de préférence enregistrées afin d'avoir un historique de la pression en cas de variation de pression.

Les valeurs de la pression Pi mesurée dans chaque ligne i (11 à 17) sont immédiatement comparées aux valeurs seuil P1 i et P3i correspondantes. Dès que dans une ligne k (par exemple 6) la valeur Pk atteint une des deux valeurs seuil P1k ou P3k, indiquant que la pression sort du domaine normal, la procédure d'analyse est mise en marche. L'instant où la pression Pk a atteint ou dépassé l'une de ces valeurs seuil P1k ou P3k est noté T1.

Dans le cas de la <u>première variante</u>, la procédure d'analyse recherche une information en provenance d'un des autres modules indiquant que le débit dans ce module à été modifié et qui pourrait expliquer cet écart de pression. Une telle information peut émaner soit directement du module, si les modifications ont été faites sur le module, soit de la base si les modifications ont été faites par son entremise. L'information peut être envoyée préventivement à tous les modules ou n'être recherchée qu'en cas de besoin par le module dont la pression est sortie du domaine normal ou par la base si elle est chargée de l'analyse.

Lorsqu'une telle information est trouvée, les paramètres du module dont la pression est sortie du domaine normal sont modifiés pour tenir compte de ce changement de débit et du changement de pression qui en découle. Si par exemple l'information indique que le débit d'un des modules du dispositif de perfusion a été diminué et que la pression Pk du module k est passée en dessous de la limite P4k, cette limite P4k au moins est revue à la baisse. De même, si l'information indique une augmentation du débit dans un module j, le paramètre P2k au moins est revu à la hausse. Les nouveaux paramètres P2k et P4k doivent cependant être à nouveau modifiés lorsque le débit dans le module j est modifié à nouveau. Par exemple, si un bolus est effectué dans le module j, la modification du débit n'est que transitoire. Par conséquent la limite P2k est augmentée (par exemple jusqu'à la valeur limite PLk pour ce module) le temps du bolus avant d'être ramenée à sa valeur initiale à la fin du bolus.

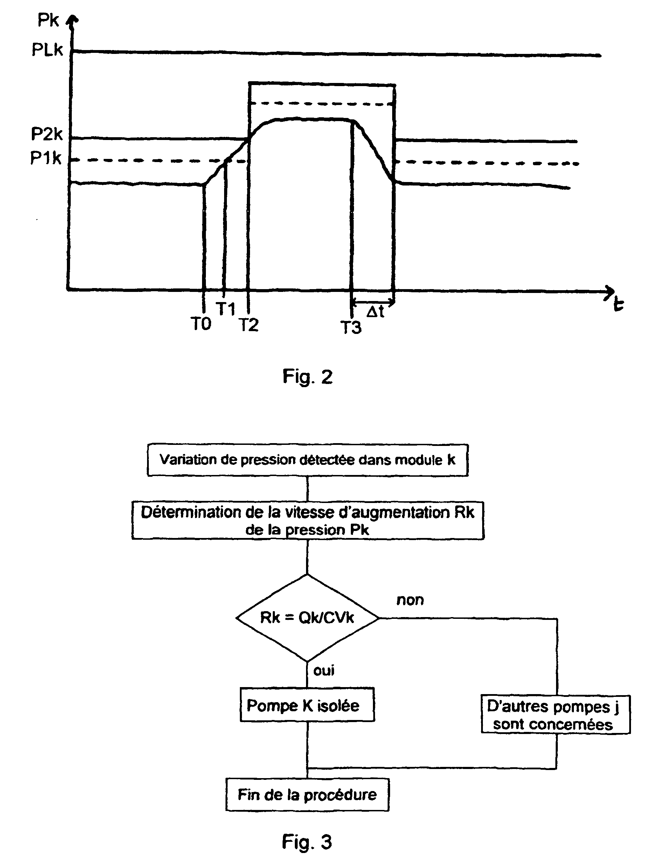

La figure 2 montre un exemple de courbe de pression dans le cas d'une augmentation de débit. Les modules j et k sont raccordés par un point de jonction (par exemple les modules (1) et (2)). On décide d'augmenter le débit de la substance administrée par le module j. A cet instant T0, la valeur limite P2j est augmentée afin que le module j ne se mette pas en alarme. La pression augmente dans la ligne j ainsi que dans la ligne k qui y est raccordée. A l'instant T1, peu après l'instant T0, la pression dans le module k atteint la limite fixée P1k et à l'instant T2 la pression Pk dépasse la valeur seuil P2k en provoquant la mise en route de la procédure d'analyse. Ayant trouvé une information en provenance du module j, indiquant que le débit dans le module j a été augmenté, le paramètre P2k est augmenté ainsi que le paramètre P1k afin de tenir compte du nouveau débit dans le module j. Lorsque le débit dans le module j est ramené à sa valeur initiale, à l'instant T3, la pression commence à diminuer, mais elle n'est pas résorbée immédiatement. Par conséquent, les valeurs P1 k et P2k ne sont ramenées à leurs valeurs initiales qu'après un laps de temps $\Delta$t.

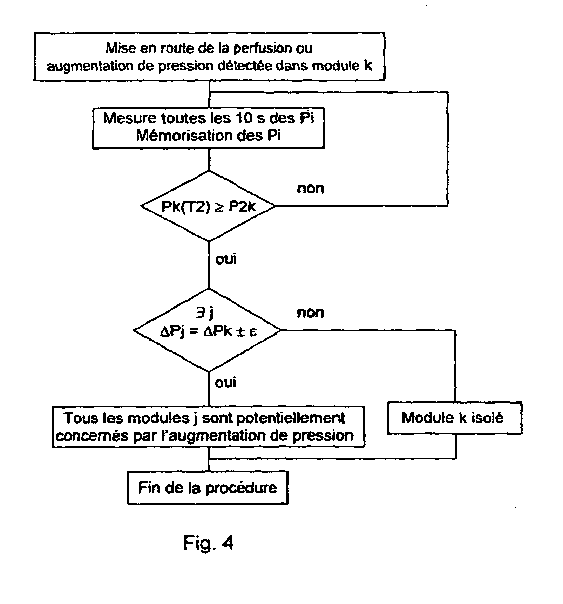

Dans la <u>seconde variante</u> de la procédure d'analyse, on cherche principalement à reconnaître les modules concernés par la variation de pression détectée dans le module k. Contrairement à la première variante, on ne tient pas compte d'informations pouvant expliquer la variation de pression. Le schéma de principe de cette variante est présenté à la figure 4.

Lorsque dans un module k la pression Pk dépasse la valeur P1 k à un instant T1, on enregistre toutes les valeurs Pi mesurées à compter de cet instant T1 (à moins que l'on ait, préventivement, enregistré systématiquement les valeurs Pi depuis le début de la perfusion). Lorsque la pression Pk dépasse la seconde valeur seuil P2k. on calcule pour chaque module i la différence de pression entre l'instant T1 et l'instant T2 :

$$\Delta Pi = Pi(T2) - Pi(T1) \qquad\qquad [1]$$

On compare ensuite les $\Delta$Pi ainsi obtenus à $\Delta$Pk. Théoriquement, toutes les lignes raccordées à la ligne k par un point de jonction devraient voir leur pression varier de la même manière que la ligne k. Dans la pratique, cette variation de pression n'est pas forcément identique en raison notamment des différences de compliance des différents modules et des moyens de mesure de la pression. On fixe donc deux domaines de tolérance caractérisés par $\varepsilon$1 et $\varepsilon$2, avec $\varepsilon$1 < $\varepsilon$2. Lorsque l'équation suivante est vérifiée :

$$\Delta Pj = \Delta Pk \pm \varepsilon 1 \qquad\qquad [2]$$

on peut en conclure que la ligne j est très vraisemblablement raccordée à la ligne k par un point de jonction.

Si par contre l'équation [2] n'est pas vérifiée mais que l'équation suivante l'est :

$$\Delta Pj = \Delta Pk \pm \varepsilon 2 \qquad\qquad [3]$$

on peut en conclure que la ligne j est probablement raccordée à la ligne k par un point de jonction.

Si même l'équation [3] n'est pas vérifiée, alors la ligne j est très vraisemblablement non raccordée à la ligne k. On fixera par exemple ε1 = 30 % et ε2 = 50 %.

Cette seconde variante de la procédure d'analyse permet donc de distinguer les lignes j concernées par la variation de pression détectée par k des autres lignes i du dispositif de perfusion qui ne le sont pas.

Si on détecte par exemple la présence d'une occlusion dans la ligne k, on peut donc en conclure que cette occlusion concerne non seulement le module et la ligne k, mais également les modules et les lignes j, tandis que les autres modules ne sont pas concernés.

En revenant à l'exemple présenté à la figure 1, si une occlusion se produit en C, et que la pression dans la ligne (7) dépasse le seuil P1(7) puis P2(7), l'analyse selon la seconde variante devrait montrer que la pression dans la ligne (6) a augmenté de façon comparable entre l'instant T1 et l'instant T2, tandis que la pression dans les autres lignes (1 à 5) est restée stable ou n'a tout du moins pas varié de manière semblable. Si par contre une rupture se produit en B et que la pression P(7) passe en dessous de la valeur seuil P3(7) puis P4(7), l'analyse selon cette seconde variante devrait montrer que la pression dans les lignes (4 à 6) a également diminué de façon comparable. Cette procédure est donc utilisable aussi bien pour une augmentation de pression que pour une diminution. Il n'est pas nécessaire que la variation de pression soit due à une occlusion ou à une rupture dans une ligne ; cette procédure peut également être mise en oeuvre lorsque la variation de pression dans la ligne k est due à une variation de débit dans une ligne j raccordée à la ligne k par un point de jonction.

Il est également possible, si les pressions sont enregistrées dès le début de la perfusion dans un fichier historique, de ne déclencher la procédure qu'au moment où Pk sort du domaine [P4k ; P2k] et de calculer a posteriori la pente de la courbe en utilisant les données du fichier historique. Si par exemple Pk dépasse le seuil d'alarme P2k à l'instant T2, on recherche dans l'historique à quel moment Pk a passé un certain seuil, par exemple P1k, et on calcule pour toutes les lignes i la pente de leurs courbes de pression entre cet instant et T2. Si par contre, pour des raisons d'économie, on préfère n'enregistrer les valeurs de pression qu'en cas de dysfonctionnement, il faudra alors lancer la procédure selon la seconde variante dès que la pression Pk sortira du domaine normal de pression [P3k ; P2k].

La <u>troisième variante</u> de la procédure d'analyse consiste principalement à déterminer si la variation de la pression détectée dans une ligne k est comparable à celle qu'elle aurait si la ligne était considérée seule dans une situation d'occlusion. Pour cela, on compare la vitesse d'augmentation de la pression. Le schéma de principe de cette variante est présenté à la figure 3.

Lorsqu'une occlusion se produit dans la ligne k, la pression Pk augmente à la vitesse Rk dépendant du débit Qk de la pompe k et de son volume de compliance CVk. Ce volume de compliance est un volume virtuel dû à l'élasticité du système lorsque la pression est supérieure à la pression normale. Dans une pompe de type pousse seringue par exemple, ce volume de compliance est dû principalement au stopper. Il se calcule à l'aide d'un coefficient de volume de compliance CVCk en fonction de la pression Pk :

$$CVk = CVCk \times Pk \qquad\qquad [4]$$

Or par définition, on a

$$Pk(t) = Rk \times t \qquad\qquad [5]$$

et

$$CVk = Ql \times t \qquad\qquad [6]$$

Par conséquent, si l'occlusion ne concerne que la pompe k (par exemple une occlusion en A ou en D), la vitesse d'augmentation de la pression en aval de la pompe k ne sera due qu'à l'apport de la pompe k et elle vaudra :

$$Rk = Qk / CVCk \qquad\qquad [7]$$

Par contre, si l'occlusion concerne d'autres pompes que la pompe k (par exemple, si l'occlusion se produit en B ou en C), la vitesse d'augmentation de la pression en aval de la pompe k dépendra du débit des autres pompes j et de leurs coefficients de volume de compliance respectifs. Elle vaudra donc :

$$Rk = \Sigma Qj / \Sigma CVCj \qquad\qquad [8]$$

Par conséquent, si on compare la vitesse d'augmentation de la pression à celle qu'on devrait obtenir si la pompe était prise isolément (équation [7]), on peut déterminer si d'autres pompes j sont concernées par l'occlusion.

Ces variantes de procédure sont basées sur des principes différents. La première variante permet de trouver dans le dispositif de perfusion une explication à une variation de pression dans un des modules. La deuxième variante permet de déterminer si des modules sont raccordés les uns aux autres. La troisième variante permet de déterminer si une occlusion est apparue et si elle concerne une ou plusieurs lignes. Il est donc intéressant de combiner ces différentes variantes pour obtenir une analyse plus fine de la situation.

Ce procédé combinant ces différentes variantes de procédure doit permettre

- de déterminer

  - s'il y a une explication acceptable à une variation de pression (augmentation ou diminution de débit dans un module),
  - si cette variation concerne d'autres modules que celui qui l'a détecté en premier et si oui lesquels

- pour ensuite agir sur le système suivant le résultat de cette analyse

  - soit en modifiant les paramètres d'analyse dans les différents modules concernés si la variation de débit à une explication acceptable,
  - soit arrêter tous les modules concernés par cette variation de débit et traiter la source du dysfonctionnement.

Lorsque dans la ligne k une variation de pression est détectée, c'est-à-dire que Pk sort du domaine normal [P3k ;P1k], la troisième variante de la procédure est tout d'abord mise en oeuvre. Si elle conduit à conclure que la vitesse d'augmentation de la pression est semblable à celle qu'on mesurerait dans la ligne si celle-ci était seule, on peut en conclure qu'une occlusion s'est produite dans la ligne k en amont de tout point de jonction avec d'autres lignes (par exemple en A ou en D). Cette conclusion serait cependant fausse si, cas exceptionnel, la ligne k était raccordée à d'autres lignes j dont les débits et les coefficients de volume de compliance respectifs étaient similaires à ceux du module k.

L'analyse se poursuit en déterminant à l'aide de la procédure d'analyse selon la seconde variante quels modules j sont concernés par cette variation de pression.

Si comme l'indique la première partie de l'analyse, seul le module k est concerné, aucun autre module ne devrait présenter de variation de pression semblable à celle détectée dans le module k. Si on se trouve dans la situation exceptionnelle évoquée plus haut, cette seconde partie de l'analyse permet de le détecter.

Une fois que l'on connaît les modules j concernés ou potentiellement concernés par cette variation de pression; la vitesse théorique d'augmentation de la pression dans la ligne k est recalculée en partant de l'hypothèse qu'une occlusion s'est formée en aval des points de jonction des lignes concernées ou potentiellement concernées et en tenant compte des débits et des coefficients de volume de compliance respectifs de ces modules j. On compare alors de nouveau la vitesse d'augmentation de la pression dans la ligne k avec cette nouvelle vitesse théorique. Si la comparaison est positive, il est très vraisemblable qu'une occlusion s'est formée en aval des points de jonction de ces lignes j. Dans le cas contraire, l'augmentation de la pression doit être due à une autre cause, par exemple un bolus dans une des lignes j. Cette hypothèse est vérifiée en recherchant selon la première variante de la procédure, s'il existe une explication à cette variation de pression.

L'ordre des variantes de procédure n'a pas d'importance. On peut bien entendu commencer par chercher une explication admissible, vérifier que le module dans lequel le débit à été modifié est bien raccordé au module k, modifier les paramètres d'analyse du module k en conséquence ainsi que ceux des modules qui sont également raccordés au module k. Si aucune explication n'est trouvée, il faut alors chercher les autres modules j concernés par la variation de pression et voir s'il s'agit d'une occlusion ou d'une rupture dans une ligne commune afin d'arrêter tous ces modules et le cas échéant mettre ces modules en marche arrière. On peut encore affiner appliquant à ces différentes variantes des coefficients de logique flou.

Une fois que la procédure d'analyse a permis de déterminer la situation exacte dans le dispositif de perfusion, il faut agir en conséquence.

Dans les dispositifs habituels, une variation de pression sortant du domaine de tolérance dans un module k conduit invariablement à une alarme et à un arrêt de la pompe en situation d'alarme. Les autres modules, y compris ceux qui sont raccordés au module k, continuent à pomper jusqu'à atteindre leurs seuils d'alarme respectifs. Le mélange en provenance des différentes lignes j va refluer sous l'effet de la pression dans la ligne k avant que les autres modules j ne soient arrêtés.

En mettant en oeuvre la procédure selon l'invention, on déterminera quels modules sont concernés par la variation de pression et on cherchera une explication à cette variation de pression. Si une explication est trouvée, les paramètres de contrôle (P1j, P2j, P3j et P4j) des modules j concernés par la variation de pression seront modifiés aussi longtemps que durera la modification ayant entraîné la variation de pression. Les modules j concernés ne seront donc pas inutilement arrêtés. Si aucune explication n'est trouvée, on en conclura qu'une occlusion ou une rupture s'est produite en aval de toutes les lignes j concernées par la variation de pression mais en amont des autres.

Une fois que l'analyse a permis de déterminer d'une part que la variation de pression n'a pas d'explication et d'autre par quels modules sont concernés, il est possible d'agir simultanément au niveau de tous les modules j concernés.

Si la variation de pression provient d'une rupture, tous les modules en amont de la rupture sont arrêtés et la rupture réparée avant que la perfusion ne soit remise en marche.

Si la variation de la pression provient d'une occlusion, tous les modules situés en amont de l'occlusion sont arrêtés simultanément. On évite ainsi le reflux d'un mélange inconnu dans la ligne k.

Afin d'éviter un bolus, il faut ramener la pression à l'intérieur des lignes j concernées à une valeur normale. Il faut donc, comme dans les dispositifs à une pompe, mettre la pompe en marche arrière pour éliminer la quantité de produit délivrée depuis l'apparition de l'occlusion. Afin d'assurer une récupération homogène des liquides délivrés, chaque pompe j concernée par l'occlusion est mise en marche arrière à un débit inverse RQj proportionnel au débit Qj initial. On déterminera parmi les pompes j la pompe s qui avait le débit Qj le plus élevé. On la mettra en marche arrière à un débit inverse RQs, par exemple le débit inverse maximum, et on mettra également les autres pompes j en marche arrière au débit inverse RQj = RQs x Qj / Qs.

La fin de l'opération de pompage inverse pourra être déterminée de différentes façons.

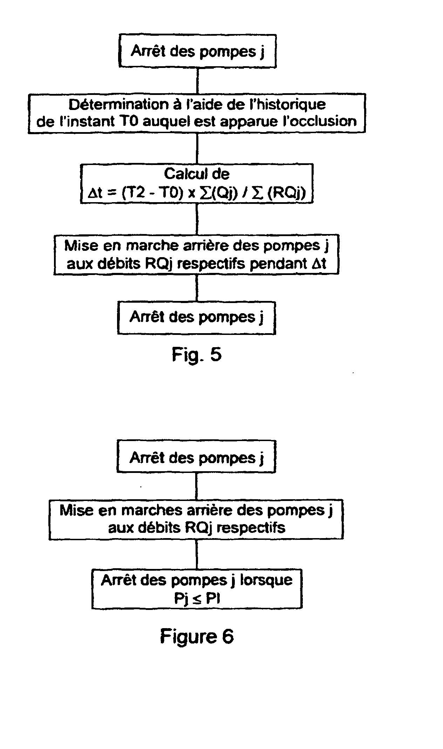

La première consiste à déterminer tout d'abord l'instant T0 auquel l'occlusion s'est produite en exploitant les données mémorisées dans l'historique. Le schéma de principe de cette solution est présenté à la figure 5. Pour cela, il faut que l'historique remonte suffisamment loin. T0 correspondra à l'intersection de la courbe de l'augmentation de la pression avec la courbe des pressions initiales stables. Les pompes j pomperont donc en marche arrière pendant une durée

$$\Delta t = (T2 - T0) \times \textstyle\sum(Qj) / \sum (RQj) = (T2 - T0) \times Qs / RQs \qquad [9]$$

Une autre solution consiste simplement à faire pomper les pompes j aux débits RQj indiqués précédemment jusqu'à ce que la pression Pj mesurée dans les tubes j repasse en dessous d'une valeur seuil PI. Le schéma de principe est présenté à la figure 6.

Lorsque la surpression est éliminée, l'occlusion peut être supprimée sans danger et le dispositif de perfusion peut être remis en marche.

Pour la mise en oeuvre du dispositif, on peut prévoir de brancher le dispositif de contrôle de chaque pompe sur un ordinateur qui dirigera les différentes opérations de contrôle.

Il est également possible de représenter sur un écran (26) placé sur la base (25) le résultat de l'analyse sous forme d'un schéma de raccordement des différentes lignes. Le personnel médical peut ainsi trouver plus facilement l'emplacement du dysfonctionnement.

Le procédé selon l'invention permet donc d'analyser la situation de l'ensemble du dispositif de perfusion. Il est donc possible d'éviter de fausses alarmes en modifiant les paramètres d'analyse si une explication acceptable est trouvée, ou au contraire d'agir directement sur l'ensemble des modules concernés par le dysfonctionnement détecté.

## Revendications

1. Procédé d'analyse de la variation de pression dans un dispositif de perfusion comprenant plusieurs modules de perfusion munis chacun d'une pompe pour entraîner un liquide à perfuser dans une ligne placée en aval de la pompe ainsi que d'un moyen de mesure de la pression dans la ligne, des points de jonction permettant de raccorder certaines lignes entre elles ou certaines lignes avec des lignes provenant d'unités externes au dispositif de perfusion, **caractérisé en ce que**, lorsqu'une variation de pression Pk dans une ligne k est détectée, une procédure d'analyse est mise en oeuvre pour déterminer l'implication d'autres modules j dans cette variation de pression.

**2.** Procédé selon la revendication 1, **caractérisé en ce que** la procédure d'analyse inclut une recherche d'informations indiquant une modification du débit dans un autre module j.

**3.** Procédé selon la revendication 2, **caractérisé en ce que** lorsqu'une information indiquant une modification de débit dans un autre module j a été trouvée, les paramètres d'analyse du module k sont modifiés au moins le temps que dure la modification du débit dans te module j.

**4.** Procédé selon l'une des revendications précédentes, **caractérisé en ce que** la procédure d'analyse inclut la comparaison de la pente de la courbe de pression de chaque ligne i du système avec la pente de la courbe de pression de la ligne k pour déterminer les lignes j qui sont potentiellement raccordées à la ligne k par un point de jonction et qui peuvent être également concernées par la variation de pression.

**5.** Procédé selon l'une des revendications précédentes, **caractérisé en ce que** la procédure d'analyse inclut la comparaison de la vitesse d'augmentation de la pression dans la ligne k avec la vitesse théorique qu'elle devrait avoir si une occlusion se produisait dans la ligne en amont de tout point de jonction avec une autre ligne.

**6.** Procédé selon les revendications 4 et 5, **caractérisé en ce que** la procédure d'analyse inclut le calcul de vitesse théorique d'augmentation de la pression qui devrait être observée dans la ligne k en cas d'occlusion en aval des points de jonction avec les lignes j et la comparaison de la vitesse d'augmentation de la pression dans la ligne k avec cette vitesse théorique.

**7.** Procédé selon l'une des revendications précédentes, **caractérisé en ce que** la pression Pi dans chaque ligne i est mesurée à intervalles réguliers, et ces mesures sont mémorisées dans un fichier historique au plus tard à partir du moment où une variation de pression est détectée dans une ligne k.

**8.** Procédé selon l'une des revendications précédentes, **caractérisé en ce que** la procédure d'analyse est lancée lorsque la pression Pk dans une ligne k atteint une valeur seuil fixée pour chaque pompe.

**9.** Procédé selon les revendications 5 ou 6, **caractérisé en ce que** lorsque les résultats de la procédure d'analyse amènent à conclure qu'une rupture ou une occlusion s'est produite en aval de la pompe k, la pompe k est arrêtée ainsi que, le cas échéant, les pompes j raccordées à la pompe k par leurs lignes respectives en des points de jonction situés en amont de la rupture ou de l'occlusion.

**10.** Procédé selon la revendication 9, **caractérisé en ce que,** lorsqu'une occlusion est détectée, chaque pompe j qui a été arrêtée est mise en marche arrière pendant un laps de temps $\Delta tj$, à un débit inverse RQj, proportionnel au débit initial Qj lors du fonctionnement normal.

**11.** Procédé selon la revendication 10, **caractérisé en ce que** les laps de temps $\Delta tj$, pendant lesquels les pompes concernées par l'occlusion pompent en marche arrière au débit inverse RQj, sont choisis identiques pour toutes lesdites pompes et valent

$$\Delta t = (T2 - T0) \times \Sigma(Qj) / \Sigma (RQj),$$

où T0 est l'instant auquel l'occlusion est apparue, cet instant T0 étant déterminé au moyen de l'historique des mesures enregistrées dès le début de la perfusion, et T2 est l'instant où la pompe k et éventuellement les pompes j ont été arrêtées.

**12.** Procédé selon la revendication 10, **caractérisé en ce que** chaque pompe j concernée pompe à l'envers jusqu'à ce que la pression déterminée dans sa ligne j soit passée en dessous d'un seuil Plj fixé.

**13.** Procédé selon l'une des revendications précédentes, **caractérisé en ce que** le résultat de la procédure d'analyse est représenté sous la forme d'un schéma de raccordement des différentes lignes.

**14.** Dispositif de perfusion comprenant plusieurs modules de perfusion munis chacun d'une pompe pour entraîner un liquide à perfuser dans une ligne placée en aval de la pompe ainsi que d'un moyen de mesure de la pression dans la ligne, des points de jonction permettant de raccorder certaines lignes entre elles ou certaines lignes avec des lignes provenant d'unités externes au dispositif de perfusion, les modules pouvant échanger des informations entre eux ou avec une base, **caractérisé en ce qu'**il est muni d'un dispositif de mise en oeuvre du procédé selon l'une des revendications précédentes.

**Claims**

1. A method for analysing a variation in pressure in a infusion device comprising several infusion modules, each fitted with a pump for flowing a liquid to be infused into a line placed downstream of the pump as well as a means for measuring the pressure in the line, junction points enabling to connect certain lines together or certain lines with lines from units external to the infusion device, **characterised in that**, in case of pressure variation Pk detected in a line k, an analysing procedure is carried out to determine the involvement of other modules j in said pressure variation.

2. A method according to claim 1, **characterised in that** the analysing procedure includes a search for information indicating a modification of the flow rate in another module j.

3. A method according to claim 2, **characterised in that** when a piece of information indicating a modification of the flow rate in another module j has been found, the analysis parameters of the module k are modified at least for the duration of the modification of the flow rate in the module j.

4. A method according to one of the previous claims, **characterised in that** the analysing procedure includes comparing the slope of the pressure curve of each line i of the system with the slope of the pressure curve of line k, to determine the lines j which are potentially connected to the line k through a junction point and which may also be affected by the pressure variation.

5. A method according to one of the previous claims, **characterised in that** the analysing procedure includes comparing the speed of pressure increase in the line k with the theoretical speed that it should have in case of occlusion in the line upstream of any junction point with another line.

6. A method according to the claims 4 and 5, **characterised in that** the analysing procedure includes the calculation of the theoretical speed of pressure increase which should be observed in the line k in case of occlusion downstream of the junction points with the lines j and comparing the speed of pressure increase in the line k with this theoretical speed.

7. A method according to one of the previous claims, **characterised in that** the pressure Pi in each line i is measured at regular intervals, and such measurements are stored in a historical file at the latest from the time of detection of a pressure variation in a line k.

8. A method according to one of the previous claims, **characterised in that** the analysing procedure is initiated when the pressure Pk in a line k reaches a threshold value set for each pump.

9. A method according to the previous claims 5 or 6, **characterised in that** when the results of the analysing procedure lead to conclude that a rupture or an occlusion has occurred downstream of the pump k, the pump k is stopped as well as, if needed, the pumps j connected to the pump k by their respective lines at junction points situated upstream of the rupture or the occlusion.

10. A method according to claim 9, **characterised in that**, when an occlusion has been detected, each pump j which has been stopped, is reversed for a time period Δtj, at a reverse flow rate RQj proportional to the initial flow rate Qj in normal operation.

11. A method according to claim 10, **characterised in that** the time periods Δtj, for which the pumps affected by the occlusion pump in reverse direction with the reverse flow rate RQj, are selected to be identical for all such pumps as follows

$$\Delta t = (T2-T0) \times \Sigma(Qj)/\Sigma(RQj),$$

where T0 is the time when the occlusion appeared, such instant T0 being determined by means of the historic of the measurements recorded from the start of the infusion, and T2 is the time when the pump k and eventually the pumps j have been stopped.

12. A method according to claim 10, **characterised in that** each pump j affected pumps in reverse direction until the pressure determined in its line j has come down below a fixed threshold Plj.

13. A method according to one of the previous claims, **characterised in that** the result of the analysing procedure is represented in the form of a wiring diagram of the different lines.

14. A infusion device comprising several infusion modules each fitted with a pump for flowing a liquid to be infused into a line placed downstream of the pump as well as measuring means of the pressure in the line, junction points enabling to connect certain lines together or certain lines with lines from units external to the infusion device, whereas the modules may exchange information together or with a base, **characterised in that** it is fitted with a device for carrying out the method according to one of the previous claims.

**Patentansprüche**

1. Verfahren zur Analyse einer Druckänderung in einer Infusionsvorrichtung mit mehreren Infusionsmodulen, die jeweils mit einer Pumpe ausgestattet sind, um eine Infusionsflüssigkeit in einer Leitung, die stromabwärts der Pumpe angeordnet ist, voranzutreiben, sowie einem Mittel zum Messen des Drucks in der Leitung, wobei Verbindungsstellen ermöglichen, bestimmte Leitungen miteinander zu verbinden oder bestimmte Leitungen mit Leitungen zu verbinden, die aus zu der Infusionsvorrichtung externen Einheiten kommen, **dadurch gekennzeichnet, dass**, wenn eine Druckänderung Pk in einer Leitung k ermittelt wird, ein Verfahren zur Analyse verwendet wird, um die Beteiligung anderer Module j an dieser Druckänderung zu bestimmen.

2. Verfahren gemäß Anspruch 1, **dadurch gekennzeichnet, dass** das Verfahren zur Analyse eine Suche nach Informationen beinhaltet, die eine Änderung des Durchsatzes in einem anderen Modul j anzeigen.

3. Verfahren gemäß Anspruch 2, **dadurch gekennzeichnet, dass**, wenn eine Information gefunden wurde, die eine Änderung des Drucks in einem anderen Modul j anzeigt, die Analyseparameter des Moduls k wenigstens für die Zeit geändert werden, die die Änderung des Durchsatzes des Moduls j dauert.

4. Verfahren gemäß einem der vorausgegangenen Ansprüche, **dadurch gekennzeichnet, dass** das Verfahren zur Analyse den Vergleich der Steigung der Druckkurve jeder Leitung i des Systems mit der Steigung der Druckkurve der Leitung k beinhaltet, um die Leitungen j zu bestimmen, die möglicherweise mit der Leitung k über eine Verbindungsstelle verbunden sind und die gleichermaßen von der Druckänderung betroffen sein können.

5. Verfahren gemäß einem der vorausgegangenen Ansprüche, **dadurch gekennzeichnet, dass** das Verfahren zur Analyse den Vergleich der Geschwindigkeit des Druckanstiegs in der Leitung k mit der theoretischen Geschwindigkeit, die diese aufweisen sollte, falls ein Verschluss in der Leitung stromaufwärts jeder Verbindungsstelle mit einer anderen Leitung entstehen würde, aufweist.

6. Verfahren gemäß Anspruch 4 und 5, **dadurch gekennzeichnet, dass** das Verfahren zur Analyse die Berechnung der theoretischen Geschwindigkeit des Druckanstiegs, die im Fall eines Verschlusses stromabwärts von Verbindungsstellen mit anderen Leitungen j in der Leitung k beobachtet werden sollte, sowie den Vergleich der Anstiegsgeschwindigkeit des Drucks in der Leitung k mit dieser theoretischen Geschwindigkeit aufweist.

7. Verfahren gemäß einem der vorausgegangenen Ansprüche, **dadurch gekennzeichnet, dass** der Druck Pi in jeder Leitung i in regelmäßigen Abständen gemessen wird, und diese Messungen in einer Verlaufsdatei spätestens ab dem Zeitpunkt, an dem eine Druckänderung in einer Leitung k ermittelt wird, gespeichert werden.

8. Verfahren gemäß einem der vorausgegangenen Ansprüche, **dadurch gekennzeichnet, dass** das Verfahren zur Analyse gestartet wird, wenn der Druck Pk in einer Leitung k einen Schwellwert, der für jede Pumpe festgelegt wird, erreicht.

9. Verfahren gemäß den Ansprüchen 5 oder 6, **dadurch gekennzeichnet, dass**, wenn die Ergebnisse des Verfahrens zur Analyse zu dem Schluss führen, dass ein Bruch oder ein Verschluss stromabwärts der Pumpe k entstanden ist, die Pumpe k sowie, falls gegeben, die Pumpen j, die mit der Pumpe k über die jeweiligen Leitungen an stromaufwärts des Bruchs oder des Verschlusses angeordneten Verbindungsstellen verbunden sind, angehalten werden.

10. Verfahren gemäß Anspruch 9, **dadurch gekennzeichnet, dass**, wenn ein Verschluss ermittelt wird, jede Pumpe j, die angehalten wurde, während einer Zeitspanne $\Delta tj$ rückwärts läuft mit einem inversen Durchsatz RQj, der zu dem Anfangsdurchsatz Qj bei einem normalen Betrieb proportional ist.

11. Verfahren gemäß Anspruch 10, **dadurch gekennzeichnet, dass** die Zeitspannen $\Delta tj$, während der die von dem Verschluss betroffenen Pumpen mit einem inversen Durchsatz RQj rückwärts laufen, für alle diese Pumpen gleich gewählt werden und den Wert

$$\Delta t = (T2 - T0) \times \sum(Qj) / \sum(RQj)$$

annehmen, bei dem T0 der Zeitpunkt ist, an dem der Verschluss aufgetreten ist, wobei der Zeitpunkt T0 mittels der Historie der seit dem Beginn der Infusion aufgezeichneten Messungen bestimmt wird, und T2 der Zeitpunkt ist, an dem die Pumpe k und gegebenenfalls die Pumpen j angehalten wurden.

12. Verfahren gemäß Anspruch 10, **dadurch gekennzeichnet, dass** jede betroffene Pumpe j rückwärts pumpt, bis der bestimmte Druck in der Leitung j unter einen festgelegten Schwellwert Plj fällt.

13. Verfahren gemäß einem der vorausgegangenen Ansprüche, **dadurch gekennzeichnet, dass** das Ergebnis des Verfahrens zur Analyse in Form eines Verbindungsschemas der unterschiedlichen Leitungen dargestellt wird.

14. Vorrichtung zur Infusion mit mehreren Infusionsmodulen, die jeweils mit einer Pumpe ausgestattet sind, um eine Infusionsflüssigkeit in einer Leitung, die stromabwärts der Pumpe angeordnet ist, voranzutreiben, sowie einem Mittel zum Messen des Drucks in der Leitung, wobei Verbindungsstellen ermöglichen, bestimmte Leitungen miteinander zu verbinden oder bestimmte Leitungen mit Leitungen zu verbinden, die aus zu der Infusionsvorrichtung externen Einheiten kommen, wobei die Module untereinander oder mit einer Basis Informationen austauschen können, **dadurch gekennzeichnet, dass** diese mit einer Vorrichtung zur Anwendung des Verfahrens gemäß einem der vorausgegangenen Ansprüche ausgestattet ist.

Fig. 1

Fig. 2

Fig. 3

Mise en route de la perfusion ou
augmentation de pression détectée dans module k

Mesure toutes les 10 s des Pi
Mémorisation des Pi

$Pk(T2) \geq P2k$    non

oui

$\exists j$
$\Delta Pj = \Delta Pk \pm \varepsilon$    non

oui

Tous les modules j sont potentiellement
concernés par l'augmentation de pression

Module k isolé

Fin de la procédure

## Fig. 4

Arrêt des pompes j

Détermination à l'aide de l'historique
de l'instant T0 auquel est apparue l'occlusion

Calcul de
$\Delta t = (T2 - T0) \times \Sigma(Qj) / \Sigma (RQj)$

Mise en marche arrière des pompes j
aux débits RQj respectifs pendant $\Delta t$

Arrêt des pompes j

Fig. 5

Arrêt des pompes j

Mise en marches arrière des pompes j
aux débits RQj respectifs

Arrêt des pompes j lorsque
$Pj \leq Pl$

Figure 6